# EUROPEAN PATENT APPLICATION

(11) **EP 3 168 670 A1**
(43) Date of publication of application: **17.05.2017**
(21) Application number: 16182444.6
(22) Date of filing: 02.08.2016
(51) Int. Cl.: G02B 21/00, C12M 3/00, G02B 21/30, H04N 5/225

(54) **MICROSCOPE UNIT AND MICROSCOPE DEVICE**

(30) Priority: 13.11.2015 TW 104137597
(71) Applicant: Aidmics Biotechnology Co., Ltd., Taipei City 10647 (TW)
(72) Inventor: LIN, Cheng-Ming, 10647 Taipei City (TW); CHEN, Chang-Yu, 10647 Taipei City (TW); CHIANG, Tsu-Chao, 10647 Taipei City (TW); LIN, Shu-Sheng, 10647 Taipei City (TW)
(74) Representative: Zimmermann, Tankred Klaus

(57) **Abstract**

A microscope unit and a microscope device are disclosed. The microscope unit is used with an external image capture module or an image capture module of the microscope device. The microscope unit comprises a body, an optical assembly and a heating element. The body has a tunnel going through the body, and a specimen observation plane located in the tunnel. The optical assembly having a convex lens is disposed at one end of the tunnel. A minimum distance from the specimen observation plane to the convex lens ranges from 0.1 mm to 3.0 mm. The heating element is disposed corresponding to the specimen observation plane.

## Description

### CROSS REFERENCE TO RELATED APPLICATIONS

This Non-provisional application claims priority under 35 U.S.C. §119(a) on Patent Application No(s). 104137597 filed in Taiwan, Republic of China on November 13, 2015, the entire contents of which are hereby incorporated by reference.

### BACKGROUND OF THE INVENTION

### Field of Invention

The invention relates to a microscope unit and a microscope device, and more particularly to a microscope unit and a microscope device capable of heating a specimen.

### Related Art

The artificial insemination (AI) technology can effectively utilize good genes to simplify the breeding operation, decrease the disease propagation and reduce the cost of feeding and managing male pigs. These advantages make the technology have the irreplaceable importance in the pig industry. The semen of one excellent male pig can inseminate a lot of female pigs, and the effectiveness thereof is several times higher than that of the natural breeding. So, the collected semen is diluted. In general, during the production process of diluting the semen in the domestic AI station, the original semen of the male pig is collected every four to six days, and only 120 to 130 milliliters of the second-stream portion with the strong sperms are collected, wherein the total sperm count ranges from 15 billions to 100 billions. The collected specimen is usually immediately mixed with the diluent having the temperature and the quantity the same as those of the collected specimen to let the sperms "adapt" to the diluent environment early, and decrease the ingredients of the semen, which can induce the unstable variation of the sperms at the same time.

In the process of waiting the temperature drop of the pre-mixed sperm to the room temperature, the basic quality test for the semen can be performed. The test contents mainly include the sperm concentration and the sperm motility. The sperm motility can be observed using a phase-contrast microscope, and is usually represented by the swimming ability of the entire sperm in the semen specimen in the form of the percentage. The swimming ability of the freshly collected original semen must range from 80 to 85% or higher. The detected pre-mixed original semen can be diluted to the final multiple using the diluent. In principle, 80 milliliters of the diluted specimen contains 15 hundred millions of total sperms (the international recommended standard ranges from 1.2 billions to 1.3 billions).

In order to lengthen the preservation time of the diluted semen, the diluted semen needs to be particularly stored in the environment of 16 *°C* (15 to 18 *°C*). However, because the male pig's sperm is very sensitive to the ambient environment temperature, the sperm motility of the specimen in the refrigerated diluted semen is seriously affected, or the test is performed on site at the diluted semen collection station. The diluted semen specimen is also affected by the weather and the temperature of the test instrument itself.

Therefore, it is an important subject to provide a test instrument with a heating function, so that the environment temperature, where the specimen is located upon testing, approaches the environment temperature, which is suitable for the specimen preservation or the insemination, and the quality of the specimen can be estimated more precisely.

### SUMMARY OF THE INVENTION

In view of the above-mentioned subjects, an objective of the invention is to provide a test instrument with a heating function, so that the environment temperature, where the specimen is located upon testing, approaches the environment temperature, which is suitable for the specimen preservation or the insemination, and the quality of the specimen can be estimated more precisely.

To achieve the above objective, the present invention discloses a microscope unit, which is used with an image capture module and includes a body, an optical assembly and a heating element. The body has a tunnel going through the body and a specimen observation plane located in the tunnel. The optical assembly has a convex lens and is disposed at one end of the tunnel. A minimum distance from the specimen observation plane to the convex lens ranges from 0.1 mm to 3.0 mm. The heating element is disposed corresponding to the specimen observation plane.

To achieve the above objective, the present invention also discloses a microscope device, which includes an image capture module and a microscope unit. The microscope unit is connected and used with the image capture module. The microscope unit includes a body, an optical assembly and a heating element. The body has a tunnel going through the body and a specimen observation plane located in the tunnel. The optical assembly has a convex lens and is disposed at one end of the tunnel. A minimum distance from the specimen observation plane to the convex lens ranges from 0.1 mm to 3.0 mm. The heating element is disposed corresponding to the specimen observation plane.

In one embodiment, the heating element is disposed on the optical assembly.

In one embodiment, the heating element is disposed on a chamber wall of the body or in the tunnel.

In one embodiment, the heating element is disposed on a chamber wall of the body or the optical assembly through a fixing member.

In one embodiment, the microscope unit has a built-in power source or the microscope unit is connected to an external power source.

In one embodiment, the heating element is an electric heating sheet, an electric heating ring, an electric heating coil or an electric heating film.

In one embodiment, the heating element is an electric heating sheet, which is a metal sheet or a glass sheet having a metal oxide coating.

In one embodiment, the image capture module is an image capture module of a mobile electronic device.

In one embodiment, the microscope device further includes a temperature sensor disposed on a chamber wall of the body.

In one embodiment, the electric heating sheet is a metal sheet or a glass sheet having a metal coating and/or a metal oxide coating, and the microscope device further includes a temperature sensor disposed on the glass sheet having the metal coating and/or the metal oxide coating.

In one embodiment, the microscope unit is electrically connected to a power source, and the microscope device further includes a control unit, which is disposed on a chamber wall of the body and is coupled to the temperature sensor and the power source. The control unit adjusts a current of the power source according to a measured temperature of the temperature sensor.

In one embodiment, the image capture module is an image capture module of a mobile electronic device.

As mentioned hereinabove, the microscope unit and the microscope device according to the invention provide the thermal energy to the specimen through the heating element, so that the environment temperature of the specimen is suitable for the specimen preservation or specimen activity, or the environment temperature for the specimen reaction approaches a temperature defined by the user.

### BRIEF DESCRIPTION OF THE DRAWINGS

The invention will become more fully understood from the detailed description and accompanying drawings, which are given for illustration only, and thus are not limitative of the present invention, and wherein:
FIG. 1 is a pictorial exterior view showing a microscope unit according to an embodiment of the invention;
FIG. 2 is an exploded view of FIG. 1;
FIG. 3 is a cross-sectional view taken along a line A-A of FIG. 1;
FIG. 4 is a pictorially cross-sectional view taken along the line A-A of FIG. 1 of the microscope unit according to another embodiment of the invention;
FIG. 5 is a pictorially cross-sectional view taken along the line A-A of FIG. 1 of a microscope unit according to still another embodiment of the invention;
FIG. 6 is a pictorially cross-sectional view taken along the line A-A of FIG. 1 of a microscope unit according to yet still another embodiment of the invention;
FIG. 7 is a pictorially cross-sectional view taken along the line A-A of FIG. 1 of a microscope unit according to yet still another embodiment of the invention;
FIG. 8 is a pictorial view showing a microscope unit connected to an external power source according to an embodiment of the invention; and
FIG. 9 is a pictorial view showing a microscope device according to an embodiment of the invention.

### DETAILED DESCRIPTION OF THE INVENTION

The present invention will be apparent from the following detailed description, which proceeds with reference to the accompanying drawings, wherein the same references relate to the same elements.

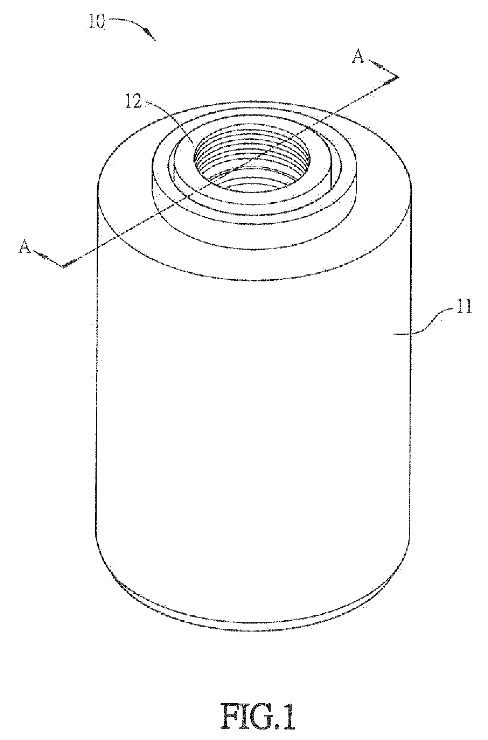

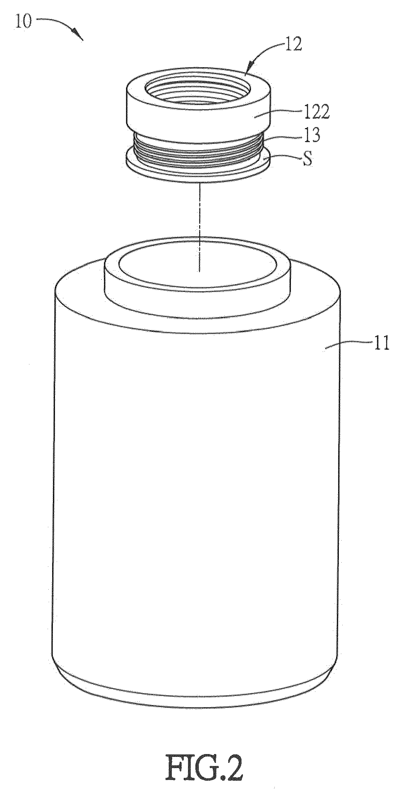

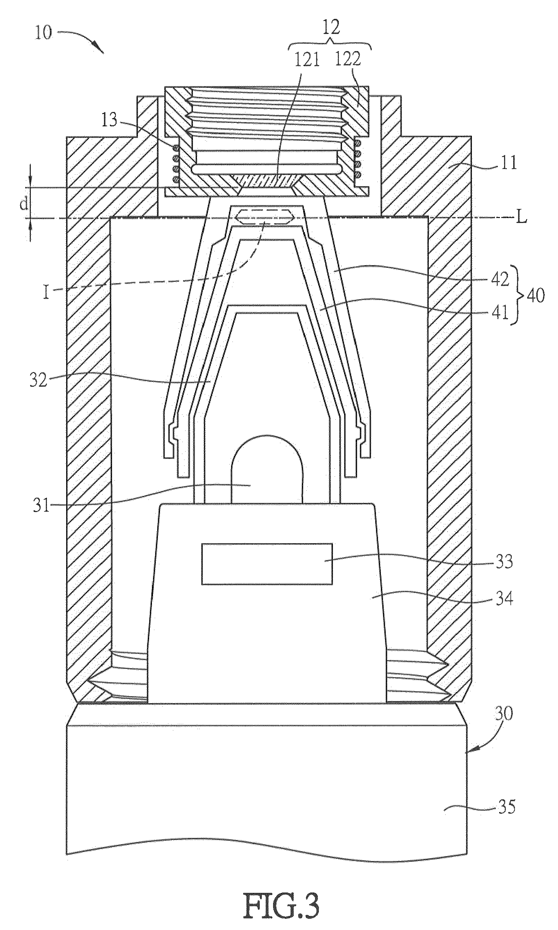

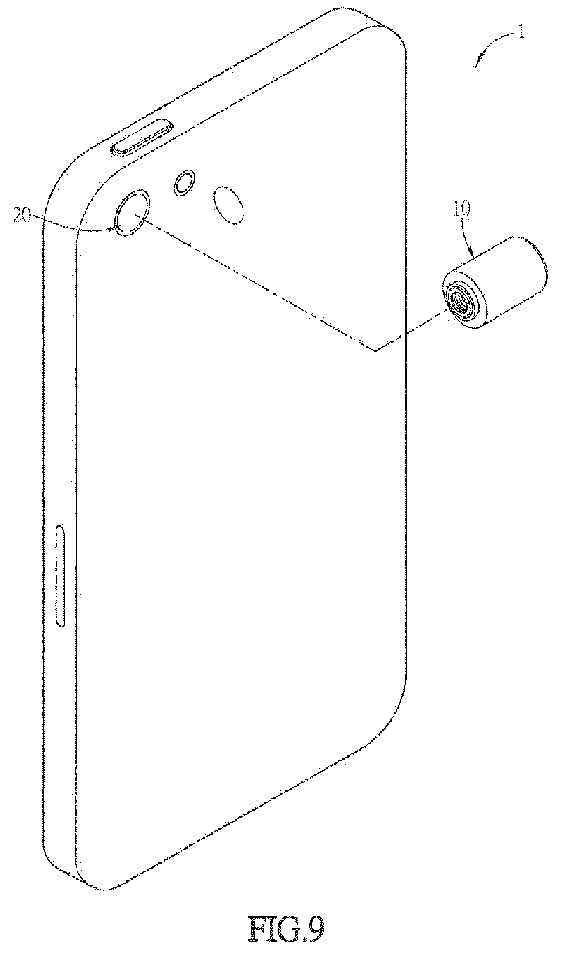

FIG. 1 is a pictorial exterior view showing a microscope unit according to an embodiment of the invention. FIG. 2 is an exploded view of FIG. 1. FIG. 3 is a cross-sectional view taken along a line A-A of FIG. 1. Referring to FIGS. 1 to 3, a microscope unit 10 is used in conjunction with an image capture module, which may be an image capture module of a mobile electronic device. The mobile electronic device may be a mobile phone, a tablet computer, a camera, an Internet Protocol (IP) camera, a driving recorder or the like, wherein the drawing of the image capture module may be found in FIG. 9. In addition, the microscope unit 10 is used with an image capture module 20 to observe the specimen, particularly the biometrics specimen, such as the tissue section, blood, semen, cell culture solution, tissue solution or the like.

The microscope unit 10 comprises a body 11, an optical assembly 12 and a heating element 13. The body 11 has a specimen observation plane L and a tunnel going through the body. The material of the body 11 may be a metallic, alloy or plastic material. The specimen observation plane L is substantially the in-focus area of the optical assembly 12, and is also the position where the specimen is placed, so that the specimen placed on the specimen observation plane L can be easily focused and the user can clearly see the zoom-in image of the specimen.

The optical assembly 12 is disposed at one end of the tunnel, and comprises a convex lens 121 and a barrel 122. The outer wall of the barrel 122 has a groove portion S, and the barrel 122 has an accommodating chamber therein. The convex lens 121 is disposed on the accommodating chamber of the barrel 122. A thread structure is formed on a top portion of the barrel 122, so that the microscope unit 10 can be screwed to the image capture module 20.

The specimen observation plane L is located in the tunnel. Because the focal length of the convex lens 121 is very short, the minimum distance d from the specimen observation plane L to the outer surface of the convex lens 121 ranges from 0.1 mm to 3.0 mm. The body 11 has a thread structure, which is formed on the other end thereof, corresponds to the optical assembly 12, and is to be screwed to a light source unit 30, which emits light to illuminate the specimen observation plane to assist in providing the backlight source to the specimen. The detailed description will be given later.

The light source unit 30 has a body 34, a light source 31, a battery 33, a light guide element 32, and a base 35. The base 35 has a flange formed with a thread structure, so that the microscope unit 10 can be screwed to the light source unit 30. The battery 33 is disposed in the body 34 of the light source unit 30, and the light source 31 is electrically connected to the battery 33 through a switch 35. The light guide element 32 has a cup-shaped structure and is flipped to fasten and cover the light source 31.

A sampling assembly 40 comprises a specimen pick-up member 41 and a specimen quantifying member 42. The specimen pick-up member 41 has a cup-shaped structure and is flipped to fasten and cover the light guide element 32. The specimen quantifying member 42 also has a cup-shaped structure, and is flipped to fasten and cover the specimen pick-up member 41 after the specimen pick-up member 41 picks up the specimen I. Thus, the specimen I is accommodated within a quantitative chamber commonly formed by the specimen pick-up member 41 and the specimen quantifying member 42.

The light source 31 provides the backlight source for the specimen and outputs the light, which is converged and illuminated onto the specimen through the light guide element 32, and diffused and reflected by and transmitted through the specimen, and then enters the convex lens 121 for imaging. Finally, the image capture module 20 captures the image, imaged by the convex lens 121, to obtain a digital image.

Please note that the heating element 13 may be an electric heating sheet, an electric heating ring, an electric heating coil or an electric heating film. The material of the heating element 13 may be a ceramic material, an alloy formed by tungsten, aluminum and copper with predetermined ratios, or a transparent electroconductive material, such as indium tin oxide (ITO). The heating element 13 is disposed corresponding to the specimen observation plane L.

For the sake of illustration, the heating element 13 of this embodiment is the electric heating coil. Referring to FIG. 3, in order to make the heating element 13 approach the to-be-observed specimen, the heating element 13 is twined and disposed in the groove portion S so as to heat the to-be-observed specimen. However, the above-mentioned configuration is used for the purpose of exemplary illustration only, and does not intend to restrict the invention. The heating element 13 may also be directly disposed in the groove portion S by way of adhering, bonding or magnetic attracting. In another embodiment, the heating element 13 may further be indirectly disposed in the groove portion through a fixing member. For example, the fixing member may be an adhesive, a tape, a lacing, a flange, a snap or a magnet.

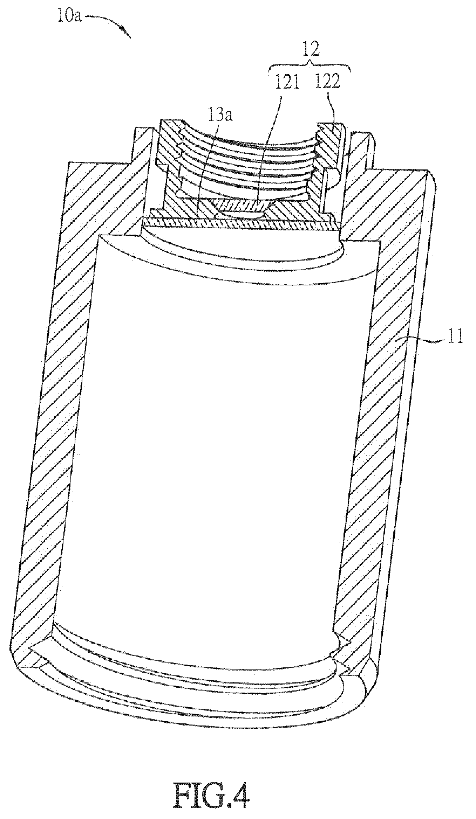

FIG. 4 is a pictorially cross-sectional view taken along the line A-A of FIG. 1 of a microscope unit 10a according to another embodiment of the invention. Referring to FIG. 4, the structure of the microscope unit 10a is substantially similar to the microscope unit 10 of the above-mentioned embodiment except for the difference that the heating element 13a of this embodiment is an electric heating sheet and is disposed under the barrel 122 of the optical assembly 12, so that the heating element 13a can be close to the specimen to provide the better heating effect to the specimen. In this embodiment, the electric heating sheet is an electric heating film composed of a layer of metal or an electroconductive transparent metal oxide, such as indium tin oxide (ITO), coated on a glass sheet. In another embodiment, however, the electric heating sheet may also be a metal sheet having the material comprising tungsten, aluminum or copper.

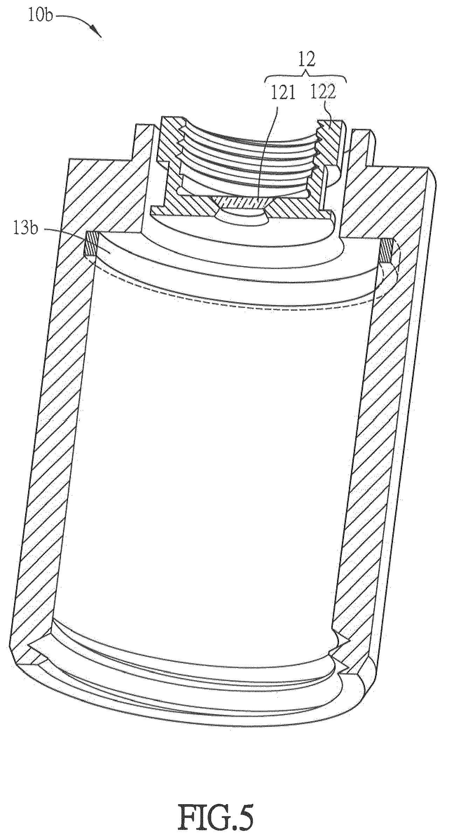

FIG. 5 is a pictorially cross-sectional view taken along the line A-A of FIG. 1 of a microscope unit according to still another embodiment of the invention. Referring to FIG. 5, the structure of the microscope unit 10b is substantially similar to the microscope unit 10 of the above-mentioned embodiment except for the difference that the heating element 13b of this embodiment corresponds to the specimen observation plane L and is disposed on the chamber wall of the body 11 and close to the specimen observation plane L. For the sake of illustration, the heating element 13b of this embodiment is an electric heating ring, and is directly embedded into the chamber wall of the body 11. However, the above-mentioned configuration is used for the purpose of exemplary illustration only, and does not intend to restrict the invention. The heating element 13b may also be directly disposed on the chamber wall of the body 11 by way of adhering, bonding or magnetic attracting. In another embodiment, the heating element 13b may further be indirectly disposed on the chamber wall of the body 11 through a fixing member. For example, the fixing member may be an adhesive, a tape, a lacing, a flange, a snap or a magnet.

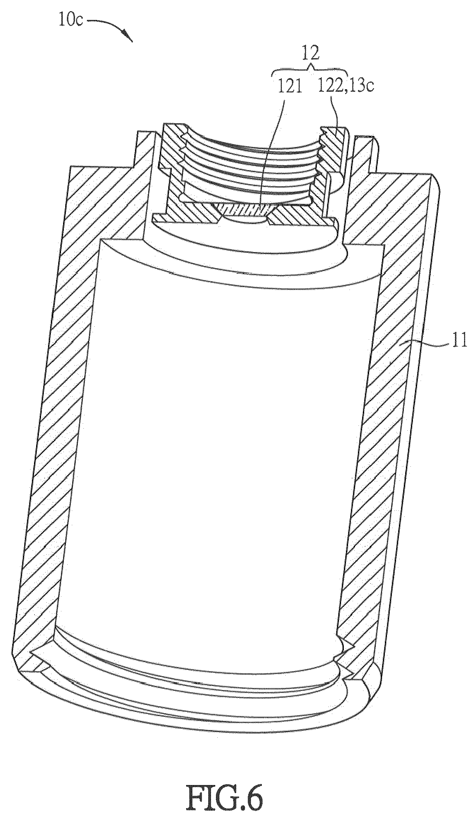

FIG. 6 is a pictorially cross-sectional view taken along the line A-A of FIG. 1 of a microscope unit according to yet still another embodiment of the invention. Referring to FIG. 6, the structure of the microscope unit 10c is substantially similar to the microscope unit 10 of the above-mentioned embodiment except for the difference that the heating element 13c of this embodiment is the barrel 122 of the optical assembly 12. That is, the material of the barrel 122 may be a ceramic material or an alloy formed by tungsten, aluminum and copper with predetermined ratios. The barrel 122 may be powered on to heat the specimen.

Please note that the spirit of the invention is to provide a temperature controlling mechanism for the to-be-observed specimen, so that the environment temperature, at which the specimen is observed, may approach the environment temperature of insemination, and the insemination ability of the semen specimen can be estimated more precisely. Thus, those skilled in the art should understand that any modification to the fixing mechanism of the heating element, which is made without departing from spirit of the invention, still falls within the scope of the invention.

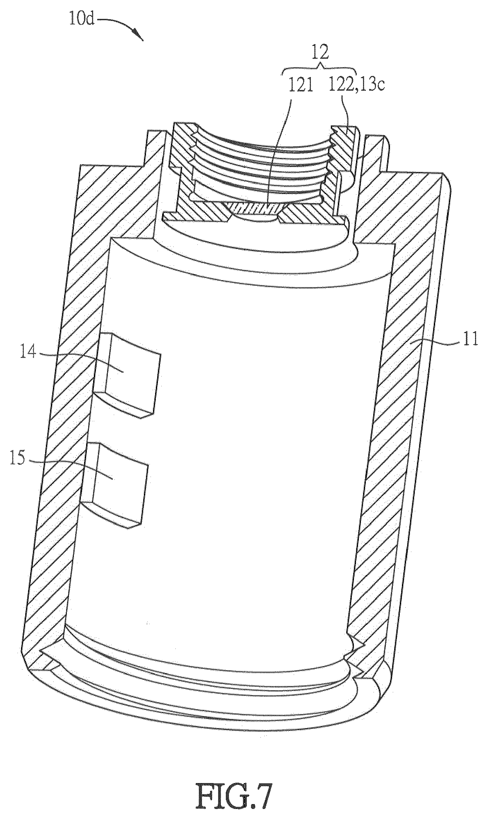

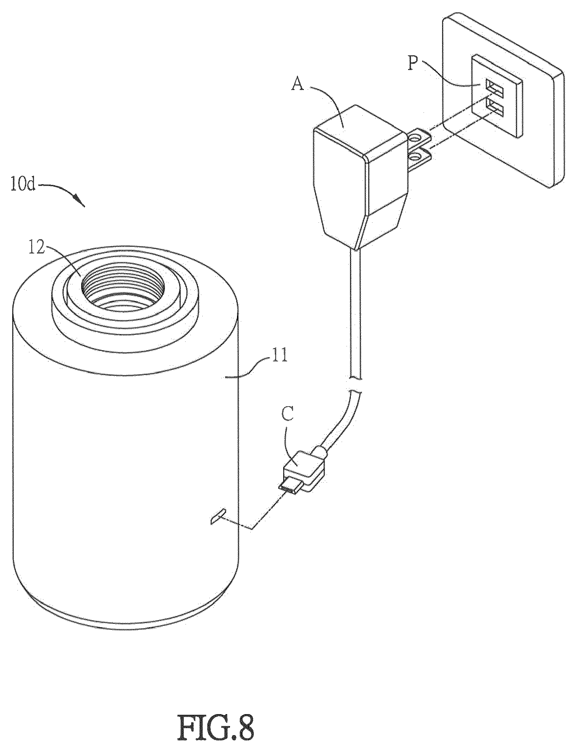

FIG. 7 is a pictorially cross-sectional view taken along the line A-A of FIG. 1 of a microscope unit according to yet still another embodiment of the invention, and FIG. 8 is a pictorial view showing a microscope unit connected to an external power source according to an embodiment of the invention. For the sake of illustration, the implementation aspect of FIG. 6 will be described. Referring to FIG. 7, the structure of the microscope unit 10a is substantially similar to the microscope unit 10 of the above-mentioned embodiment except for the difference that the structure of the microscope unit 10d is substantially similar to that of the microscope unit 10c of FIG. 6 except for the difference that the microscope unit 10d further comprises a temperature sensor 14 and a control circuit 15. The temperature sensor 14 is electrically connected to the control circuit 15, which is electrically connected to a current source P. The current source P is electrically connected to the heating element 13c through a transformer A and a USB connector C. The current source P provides a current to the heating element 13c so that the heating element 13c generates the thermal energy. The temperature sensor 14 is for measuring the temperature in the chamber of the body 11, and transfers the temperature in the chamber of the body 11 to the control circuit 15. The control circuit 15 adjusts the current provided by the current source P according to the measured temperature of the temperature sensor 14 to achieve the temperature controlling function. Please note that the current source P in this embodiment is an external power source inputted to the microscope unit 10d through the USB interface. However, the invention is not restricted thereto. The battery 33 of the light source unit 30 may also supply the current to the heating element 13c.

In addition, the temperature sensor 14 and the control circuit 15 are disposed on the chamber wall of the body 11 in this embodiment. In another embodiment, however, the control circuit 15 may also be independently disposed outside the body 11. For example, the control circuit 15 is integrated into the transformer A, the USB connector C, or a connector (not shown) disposed between the USB connector C and the microscope unit 10. In still another embodiment, the body 11 may be a hollow barrel, in which the control circuit 15 may be disposed. Any modification made by those skilled in the art without departing from spirit of the invention still falls within the scope of the invention

In addition, the temperature sensor 14 and the control circuit 15 may also be integrated into the microscope unit 10, 10a, 10b or 10c. After reviewing the above-mentioned paragraphs, those skilled in the art may modify the aspect of the microscope unit 10, 10a, 10b or 10c, so that the temperature sensor 14 is used with the control circuit 15. For example, the temperature sensor 14 may be disposed on the ITO glass of the heating element 13a in the microscope unit 10a. For the sake of conciseness, detailed descriptions of the modified aspect where the temperature sensor 14 and the control circuit 15 are integrated into the microscope unit 10, 10a, 10b or 10c will be omitted.

FIG. 9 is a pictorial view showing a microscope device according to an embodiment of the invention. In FIG. 9, a microscope device 1 comprises a microscope unit 10 and an image capture module 20. The microscope unit 10 may be any aspect of the microscope units 10a, 10b, 10c and 10d, wherein the detailed operation principles and connection structures may be found thereabove, and will be omitted for the sake of conciseness. After reviewing the above-mentioned paragraphs, those skilled in the art can modify the microscope device 1 according to the aspect of the microscope unit 10a, 10b, 10c or 10d. The image capture module 20 is an image capture module of a mobile electronic device. In this embodiment, the mobile electronic device is a mobile phone, which does not intend to restrict the invention. The mobile electronic device may also be a tablet computer, a camera, an IP camera, a driving recorder or the like.

In addition, in order to make the heat, generated by the heating element 13, be uniformly transferred to the tunnel of the body 11, the microscope unit 10 of the invention may further comprise a heating aid, which may be made of a thermoconductive material, such as silver, copper or an alloy thereof. For example, the heating aid may be a metal sleeve disposed in the tunnel of the body 11, and the heating element 13 is twined or covers the outer wall of the heating aid. In other words, the heating element 13 is located in the tunnel, and more clearly located between the outer wall of the heating aid and the inner wall of the tunnel of the body 11. The heating aid coupled to the heating element 13 receives the heat generated by the heating element 13, and then uniformly transfers the received heat to the overall tunnel. Please note that the heating aid may also be applied to the microscope unit 10a, 10b, 10c or 10d, and the invention is not restricted thereto. In another embodiment, the body 11 may be a hollow barrel, and the control circuit 15 may be thus disposed therein.

In summary, the microscope unit and the microscope device according to the invention provide the thermal energy to the specimen through the heating element, so that the environment temperature of the specimen is suitable for the specimen preservation or specimen activity, or the environment temperature for the specimen reaction approaches a temperature defined by the user.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A microscope unit used with an image capture module, the microscope unit comprising:
a body having a tunnel going through the body, and a specimen observation plane located in the tunnel;
an optical assembly, which has a convex lens and is disposed at one end of the tunnel, wherein a minimum distance from the specimen observation plane to the convex lens ranges from 0.1 mm to 3.0 mm; and
a heating element disposed corresponding to the specimen observation plane.

2. The microscope unit according to claim 1, wherein the heating element is disposed on the optical assembly.

3. The microscope unit according to claim 1, wherein the heating element is disposed on a chamber wall of the body or in the tunnel.

4. The microscope unit according to claim 1, wherein the heating element is disposed on a chamber wall of the body or the optical assembly through a fixing member.

5. The microscope unit according to claim 1, wherein the heating element covers the convex lens.

6. The microscope unit according to claim 1, wherein the microscope unit has a built-in power source or the microscope unit is connected to an external power source.

7. The microscope unit according to claim 1, wherein the heating element is an electric heating sheet, an electric heating ring, an electric heating coil or an electric heating film.

8. The microscope unit according to claim 1, wherein the image capture module is an image capture module of a mobile electronic device.

9. A microscope device, comprising:
an image capture module; and
a microscope unit connected to the image capture module, the microscope unit comprising:
a body having a tunnel going through the body, and a specimen observation plane located in the tunnel;
an optical assembly, which has a convex lens and is disposed at one end of the tunnel, wherein a minimum distance from the specimen observation plane to the convex lens ranges from 0.1 mm to 3.0 mm; and
a heating element disposed corresponding to the specimen observation plane.

10. The microscope device according to claim 9, wherein the heating element is disposed on the optical assembly.

11. The microscope device according to claim 9, wherein the heating element is disposed on a chamber wall of the body or in the tunnel.

12. The microscope device according to claim 9, wherein the heating element is disposed on a chamber wall of the body or the optical assembly through a fixing member.

13. The microscope device according to claim 9, wherein the heating element covers the convex lens.

14. The microscope device according to claim 9, wherein the heating element is an electric heating sheet, an electric heating ring, an electric heating coil or an electric heating film.

15. The microscope device according to claim 14, wherein the electric heating sheet is a metal sheet or a glass sheet having a metal coating and/or a metal oxide coating, and the microscope device further comprises:
a temperature sensor disposed on a chamber wall of the body, or the glass sheet having the metal coating and/or the metal oxide coating.
